**Europäisches Patentamt**

**European Patent Office**

**Office européen des brevets**

(19)

(11) Veröffentlichungsnummer: **0 071 080**
**B1**

# EUROPÄISCHE PATENTSCHRIFT

(12)

(45) Veröffentlichungstag der Patentschrift:
**25.09.85**

(21) Anmeldenummer: **82106283.3**

(22) Anmeldetag: **14.07.82**

(51) Int. Cl.⁴: **C 07 C 118/00**, C 07 C 119/042,
C 08 G 18/10, C 08 G 18/76 //
C07C118/02

(54) **Verfahren zur Herstellung von Polyisocyanaten.**

(30) Priorität: **24.07.81 DE 3129270**

(43) Veröffentlichungstag der Anmeldung:
**09.02.83 Patentblatt 83/6**

(45) Bekanntmachung des Hinweises auf die Patenterteilung:
**25.09.85 Patentblatt 85/39**

(84) Benannte Vertragsstaaten:
**BE DE FR GB IT NL**

(56) Entgegenhaltungen:
**DE - B - 1 593 412**

(73) Patentinhaber: **BAYER AG, Konzernverwaltung RP
Patentabteilung, D-5090 Leverkusen 1 Bayerwerk (DE)**

(72) Erfinder: **Wegener, Gerhard, Dr., Futterstrasse 33,
D-5600 Wuppertal 2 (DE)**
Erfinder: **Knöfel, Hartmut, Dr., Dülmener Weg 21,
D-5060 Odenthal (DE)**
Erfinder: **Ellendt, Günther, Dr., Deswatinesstrasse 81,
D-4150 Krefeld (DE)**
Erfinder: **Petinaux, Marcel, Dr., Flensburger Zeile 1b,
D-4150 Krefeld (DE)**

**Beschreibung**

Die Erfindung betrifft ein neues Verfahren zur Herstellung von organischen Polyisocyanaten in Gegenwart von Lösungsmitteln und unter Wiederverwendung des eingesetzten Lösungsmittels, wobei man das Lösungsmittel vor seiner Wiederverwendung zumindest teilweise mit Verbindungen mit gegenüber Isocyanatgruppen reaktionsfähigen Wasserstoffatomen behandelt und gegebenenfalls anschliessend destillativ aufarbeitet.

Organische Polyisocyanate werden grosstechnisch durch Phosgenierung der ihnen entsprechenden primären Polyamine hergestellt, wobei man die Phosgenierung in Gegenwart von inerten organischen Lösungsmitteln wie z.B. Chlorbenzol oder ortho-Dichlorbenzol durchführt (vgl. z.B. Ullmanns Encyklopädie der technischen Chemie, 4. Auflage (1977), Band 13, Seiten 350 ff.).

Bei der Herstellung der technisch wichtigen Polyisocyanate, insbesondere bei der Herstellung von Hexamethylendiisocyanat, Toluylendiisocyanaten und Polyisocyanaten der Diphenylmethanreihe durch Phosgenierung der entsprechenden Di- und Polyamine entstehen stets Spuren an Isocyanatgruppen aufweisenden Verbindungen (z.B. 6-Chlor-hexylisocyanat im Falle der Herstellung von Hexamethylendiisocyanat , Tolylisocyanat im Falle der Herstellung von Toluylendiisocyanaten und Phenylisocyanat im Falle der Herstellung von Polyisocyanaten der Diphenylmethanreihe durch Phosgenierung von Anilin/Formaldehyd-Kondensaten). Zur Verringerung des Gehalts der Verfahrensprodukte (Polyisocyanate) an derartigen, Isocyanatgruppen aufweisenden, die Qualität der Verfahrensprodukte äusserst negativ beeinflussenden Verbindungen war man bislang bestrebt, diese nach der Phosgenierungsreaktion zusammen mit dem Lösungsmittel destillativ aus dem Polyisocyanat zu entfernen und das Lösungsmittel seinerseits vor seiner Wiederverwendung durch eine aufwendige Kolonnendestillation von den Verunreinigungen zu befreien. Diese destillative Aufarbeitung des Lösungsmittels ist nur mit einem hohen Energie- und apparativen Aufwand möglich, wobei insbesondere diejenigen Verbindungen Schwierigkeiten bereiten, deren Siedepunkte in der Nähe der Siedepunkte der jeweiligen Lösungsmittel liegen.

Es war die der vorliegenden Erfindung zugrundeliegende Aufgabe, ein neues Verfahren zur Herstellung von Polyisocyanaten unter Wiederverwendung der als Reaktionsmedium dienenden Lösungsmittel zur Verfügung zu stellen, welches einerseits die Herstellung von Polyisocyanaten mit einem stark erniedrigten Gehalt an Isocyanatgruppen aufweisenden Nebenkomponenten, insbesondere an Monoisocyanaten gestattet, und welches andererseits aufwendige Kolonnendestillationen zwecks Aufarbeitung des wiederzuverwendenden Lösungsmittels überflüssig macht.

Diese Aufgabe konnte mit dem nachstehend näher beschriebenen erfindungsgemässen Verfahren gelöst werden.

Gegenstand der Erfindung ist ein Verfahren zur Herstellung von Polyisocyanaten durch

a) Umsetzung von (i) Lösungen der den Polyisocyanaten zugrundeliegenden Polyamine in einem inerten Lösungsmittel mit (ii) einer Lösung von Phosgen in einem inerten Lösungsmittel in einer ein- oder mehrstufigen Phosgenierungsreaktion,

b) Abtrennung des überschüssigen Phosgens und des gebildeten Chlorwasserstoffs aus dem gemäss a) erhaltenen flüssigen Reaktionsgemisch,

c) Abtrennung des Lösungsmittels zusammen mit leicht flüchtigen, NCO-Gruppen tragenden Verbindungen aus der gemäss b) erhaltenen Lösung durch eine Verdampfung und Gewinnung des gegebenenfalls einer weiteren destillativen Aufarbeitung zuzuführenden Verfahrensprodukts als Verdampfungsrückstand,

d) Gewinnung eines flüchtige, NCO-Gruppen tragende Verbindung enthaltenden Lösungsmittels durch Kondensation der gemäss c) anfallenden Dämpfe und Wiederverwendung eines Teils des Kondensats zur Herstellung von Aminlösung (i) und eines zweiten Teils des Kondensats zur Herstellung von Phosgenlösung (ii),
dadurch gekennzeichnet, dass man vor der Wiederverwendung des Kondensats entweder

e) die Gesamtmenge des gemäss d) erhaltenen Kondensats ohne vorhergehende destillative Aufarbeitung mit Verbindungen mit gegenüber Isocyanatgruppen reaktionsfähigen Wasserstoffatomen, die mit dem Lösungsmittel keine Azeotrop bilden, die für die destillative Abtrennung ausreichend über sieden als das Lösungsmittel selbst, und die ausgewählt sind aus der Gruppe bestehend aus ein- und mehrwertigen Alkoholen, mono- und polyfunktionellen primären und sekundären Aminen und Gemischen derartiger Verbindungen behandelt und anschliessend destillativ von schwerflüchtigen, durch Reaktion dieser Verbindungen mit gegenüber Isocyanatgruppen reaktionsfähigen Wasserstoffatomen mit im Kondensat enthaltenen, Isocyanatgruppen aufweisenden Verbindungen gebildeten, Reaktionsprodukten befreit, oder

f) die aus undestilliertem, gemäss d) erhaltenem Kondensat bestehende, zur Herstellung der Aminlösung (i) verwendete Teilmenge des Lösungsmittels mit Verbindungen mit gegenüber Isocyanatgruppen reaktionsfähigen Wasserstoffatomen der unter e) genannten Art behandelt und gegebenenfalls anschliessend das so behandelte Lösungsmittel destillativ von schwerflüchtigen, durch Reaktion dieser Verbindungen mit gegenüber Isocyanatgruppen reaktionsfähigen Wasserstoffatomen mit den Isocyanatgruppen aufweisenden Verbindungen gebildeten, Reaktionsprodukten befreit.

Die Phosgenierungsreaktion a) erfolgt in an sich bekannter Weise unter Verwendung von (i) Lösungen von Polyaminen in inerten Lösungsmitteln und Phosgenlösungen in inerten Lösungsmitteln, wobei auch beim erfindungsgemässen Verfahren die Phosgenierungsreaktion ein- oder mehrstufig (beispielsweise Bildung von Suspensionen von Carbamidsäurechloriden in der Kälte und anschliessende Überführung dieser Suspension in Polyisocyanatlösungen in der Hitze «Kalt-/Heissphosgenierung») erfolgen kann. Für die Stufe a) des erfindungsgemässen Verfahrens sind insbesondere die technisch wichtigen Polyamine wie beispielsweise Hexamethylendiamin, 2,4- und/oder 2,6-Diaminotoluol, 2,4'- und 4,4'-Diaminodiphenylmethan sowie deren als «Polyamingemische der Diphenylmethanreihe» zu bezeichnenden Gemische mit höheren Homologen, wie sie durch Anilin/Formaldehyd-Kondensation in an sich bekannter Weise zugänglich sind, 1,5-Diaminonaphthalin, 1-Amino-3,3,5-trimethyl-5-aminomethyl-cyclohexan (Isophorondiamin), Tris-(isocyanatophenyl-)methan oder perhydrierte Diaminodiphenylmethane geeignet.

Bei der Durchführung des erfindungsgemässen Verfahrens kommen die beispielhaft genannten Amine in Form von 3- bis 40-gew.-%igen, vorzugsweise 5- bis 25-gew.-%igen Lösungen in inerten Lösungsmitteln zum Einsatz.

Das bei der Phosgenierungsreaktion zum Einsatz gelangende Phosgen seinerseits kommt in Form von 10- bis 60-gew.-%igen, vorzugsweise 25- bis 50-gew.-%igen Lösungen in inerten Lösungsmitteln zum Einsatz.

Geeignete inerte Lösungsmittel sowohl für das Polyamin als auch für das Phosgen sind beispielsweise Chlorbenzol oder ortho-Dichlorbenzol.

Nach der gemäss Verfahren des Standes der Technik durchgeführten Reaktionsstufe a) wird das überschüssige Phosgen und der gebildete Chlorwasserstoff beispielsweise durch Ausblasen mit Inertgas oder durch Andestillieren in der Reaktionsstufe b) entsprechend den bekannten Verfahren des Standes der Technik entfernt.

In der Stufe c) des erfindungsgemässen Verfahrens wird das in Form einer Lösung vorliegende Phosgenierungsprodukt durch einfaches Abdampfen in eine, flüchtige NCO-Gruppen tragende Verbindungen enthaltende, Gasphase und eine im wesentlichen aus rohem Polyisocyanat bestehende Flüssigphase getrennt. Die hierbei anfallende flüssige Phase kann gewünschtenfalls zwecks Reindarstellung des Polyisocyanats in an sich bekannter Weise destillativ aufgearbeitet werden. Die Stufe c) des erfindungsgemässen Verfahrens wird im allgemeinen bei einer Temperatur von 80 bis 220°C, vorzugsweise 120 bis 190°C bei einem Druck von 100 bis 4000, vorzugsweise 200 bis 3000 mbar durchgeführt.

Bei der Stufe d) des erfindungsgemässen Verfahrens werden die gemäss c) anfallenden Dämpfe zu einem, flüchtige insbesondere Monoisocyanate enthaltenden Lösungsmittel-Kondensat kondensiert. Der Gehalt des Kondensats an den genannten Verbindungen (berechnet als NCO mit dem Molekulargewicht 42) liegt im allgemeinen bei 50 bis 5000, insbesondere 100 bis 1500 ppm.

Das Lösungsmittel wird nun zur erneuten Herstellung von Amin- und Phosgenlösungen wiederverwendet. Im Unterschied zu den bekannten Verfahren des Standes der Technik tritt jedoch an die Stelle einer aufwendigen Kolonnendestillation zwecks Beseitigung der Isocyanatgruppen aufweisenden Verbindungen eine der nachstehend näher beschriebenen erfindungsgemässen Massnahmen.

Gemäss der ersten Ausführungsform des erfindungsgemässen Verfahrens wird das gemäss d) erhaltene Kondensat vor seiner Auftrennung in zur Herstellung von Aminlösung (i) und Phosgenlösung (ii) bestimmte Teilströme mit einer Verbindung mit gegenüber Isocyanatgruppen reaktionsfähigen Wasserstoffatomen behandelt und anschliessend destillativ von schwerflüchtigen, durch Reaktion dieser Verbindungen mit gegenüber Isocyanatgruppen reaktionsfähigen Wasserstoffatomen mit den Isocyanatgruppen aufweisenden Verbindungen gebildeten, Reaktionsprodukten befreit. Die genannte Umsetzung und destillative Abtrennung des reinen Lösungsmittels kann sowohl ein- als auch mehrstufig erfolgen und zwar entweder dergestalt, dass man die Umsetzung in der zur Destillation verwendeten Destillationskolonne selbst oder in einer der Destillationsstufe vorgeschalteten Reaktionsstufe durchführt. Durch die erfindungsgemässe Behandlung des gemäss d) erhaltenen Kondensats werden im Kondensat enthaltene leichtflüchtige, Isocyanatgruppen aufweisende Verbindungen in schwerflüchtige Reaktionsprodukte überführt, die bei der anschliessenden destillativen Abtrennung des Lösungsmittels einen weit geringeren Destillationsaufwand erfordern. Während zur Abtrennung der leichtflüchtigen, Isocyanatgruppen aufweisenden Verbindungen eine aufwendige fraktionierte Destillation erforderlich ist, können die schwerflüchtigen Reaktionsprodukte ohne grösseren Aufwand im Sumpf der Destillationsapparatur angereichert und ausgeschleust werden.

Im allgemeinen werden als Verbindungen mit gegenüber Isocyanatgruppen reaktionsfähigen Wasserstoffatomen solche eingesetzt, die mit dem Lösungsmittel kein Azeotrop bilden, die für die destillative Abtrennung ausreichend höher sieden als das Lösungsmittel selbst, und die ausgewählt sind aus der Gruppe bestehend aus ein- und mehrwertigen Alkoholen, vorzugsweise mit primären Hydroxylgruppen, mono- und polyfunktionellen primären und sekundären Aminen und Gemischen aus beliebigen derartigen Verbindungen. Vorzugsweise werden derartige Verbindungen eingesetzt, die mindestens 2 gegenüber Isocyanatgruppen reaktionsfähige Gruppen aufweisen. Die Verbindungen können auch in Form von Lösungen in einem Lösungsmittel der jeweils zu behandelten Art eingesetzt werden. Typische Beispiele derartiger Verbindungen sind Butandiol-1,3, Butandiol-1,4, Hexandiol-1,6, 2,2,5-Trimethyl-hexandiol-1,6, Diethylenglykol, Triethylenglykol, Tetraethylenglykol, Glycerin, Trimethylolpropan, n-Octadecanol, Anilin, 2,2'-, 2,4- und 4,4'-Diaminodiphenylmethan, Gemische dieser Isomeren mit höheren Homologen, wie sie durch Anilin/Formaldehyd-Kondensation in bekannter Weise erhalten werden, 2,4- und/oder 2,6-Diaminotoluol, 1-Aminonaphthalin, 1,5-Diamino-naphthalin, Tetramethylendiamin, Hexamethylendiamin, Isophorondiamin oder 4,4'-Diamino-dicyclohexylmethan.

Die Behandlung des gemäss d) erhaltenen Kondensats mit den beispielhaft genannten Verbindungen mit gegenüber Isocyanatgruppen reaktionsfähigen Wasserstoffatomen erfolgt im allgemeinen im Temperaturbereich von 80 bis 200°C, vorzugsweise 100 bis 160°C und insbesondere zwischen 120 und 155°C. Im Interesse einer möglichst quantitativen Entfernung der Isocyanatgruppen aufweisenden Verbindungen ist es zweckmässig, die genannten erfindungsgemässen Reaktionspartner mit gegenüber Isocyanatgruppen reaktionsfähigen Wasserstoffatomen, bezogen auf die Isocyanatgruppen der im Kondensat gemäss d) enthaltenen Verbindungen in mindestens äquivalenten Mengen einzusetzen. Da vorzugsweise als Verbindungen mit gegenüber Isocyanatgruppen reaktionsfähigen Wasserstoffatomen solche eingesetzt werden, die aufgrund ihres höheren Siedepunktes leicht vom Lösungsmittel destillativ abgetrennt werden können, ist es durchaus auch möglich, einen Überschuss dieser Verbindungen einzusetzen. Die Verwendung eines Unterschusses würde selbstverständlich lediglich zu einer teilweisen Entfernung der NCO-Gruppen tragenden Verbindungen führen. Im allgemeinen liegt das Äquivalentverhältnis zwischen Isocyanatgruppen der aus dem Lösungsmittel abzutrennenden Verbindungen und den gegenüber Isocyanatgruppen reaktionsfähigen Gruppen (Hydroxyl- und/oder Aminogruppen) während der Umsetzung bei 1 : 1 bis 1 : 2.

Grundsätzlich ist es auch möglich, bei der ersten Ausführungsform des erfindungsgemässen Verfahrens solche Verbindungen mit gegenüber Isocyanatgruppen reaktionsfähigen Wasserstoffatomen einzusetzen, die einen niedrigeren Siedepunkt als das aufzuarbeitende Lösungsmittel aufweisen. Auch derartige Verbindungen, wie z.B. Methanol, Ethanol, Ethylamin oder n-Hexylamin führen durch Reaktion mit den Isocyanatgruppen aufweisenden Verbindungen zu Reaktionsprodukten, die einen höheren Siedepunkt als das aufzuarbeitende Lösungsmittel aufweisen. Da diese Verbindungen mit gegenüber Isocyanatgruppen reaktionsfähigen Wasserstoffatomen selbst jedoch einen tieferen Siedepunkt als das aufzuarbeitende Lösungsmittel aufweisen und daher nicht zusammen mit den Umsetzungsprodukten der erfindungsgemässen Behandlung im Destillationssumpf anfallen, können sie lediglich in unteräquivalenten bis max. äquivalenten Mengen zum Einsatz gelangen, was selbstverständlich auf eine nur teilweise Entfernung der Isocyanatgruppen aufweisenden Verbindungen hinauslaufen würde.

Die erfindungsgemässe Behandlung des gemäss d) erhaltenen Lösungsmittelkondensats mit den Verbindungen mit gegenüber Isocyanatgruppen reaktionsfähigen Wasserstoffatomen wird, wie dargelegt, entweder in der Destillationsstufe selbst oder einer der Destillationsstufe vorgeschalteten Reaktionsstufe durchgeführt. Die Behandlung ist innerhalb der genannten Temperaturbereiche im allgemeinen nach einer mittleren Verweilzeit von 5 bis 60 min beendet, wobei die beispielhaft genannten primären Amine wegen ihrer höheren Reaktionsbereitschaft gegenüber Isocyanatgruppen selbstverständlich eine geringere Verweilzeit als die Alkohole erfordern.

Die im Laufe der erfindungsgemässen Behandlung gebildeten schwerflüchtigen Reaktionsprodukte werden gemäss erster Ausführungsform des erfindungsgemässen Verfahrens im allgemeinen in Form von 5- bis 95-gew.-%igen, vorzugsweise 20- bis 85-gew.-%igen und insbesondere 35- bis 60-gew.-%igen, im Sumpf der Destillationsstufe anfallenden Konzentraten aus dem Destillationssumpf ausgetragen.

Das als Destillat anfallende, von den NCO-Gruppen tragenden Verbindungen befreite Lösungsmittel wird dann erneut zur Herstellung von Aminlösung (i) und Phosgenlösung (ii) eingesetzt. Auf diese Weise wird erreicht, dass bei der Durchführung des erfindungsgemässen Verfahrens gemäss erster Ausführungsform Polyisocyanate mit einem stark reduzierten Gehalt an leichtflüchtigen Isocyanatkomponenten entstehen. Gleichzeitig wird der Energie- und apparative Aufwand bezüglich der durchzuführenden Destillationsschritte ganz wesentlich reduziert.

Darüber hinaus besitzt das erfindungsgemässe Verfahren, insbesondere die erste Ausführungsform und die erste Variante der zweiten Ausführungsform, gegenüber dem Verfahren des Standes der Technik den weiteren Vorteil, dass die zur Phosgenierung eingesetzten Amine einen höheren Anteil an Nebenkomponenten, — z.B. bedingt durch die Art ihrer Herstellung — enthalten können, welche bei der Phosgenierung zu einem entsprechend erhöhten Anteil an erfindungsgemäss abtrennbaren, NCO-Gruppen tragenden Verbindungen führen, als das bei den nach dem derzeitigen Stand der Technik eingesetzten Aminen toleriert werden kann. Daraus resultiert bereits auf der Stufe der Amine eine beträchtliche Verringerung des apparativen und energiemässigen Aufwandes, der bei deren Vorbereitung gemäss dem derzeitigen Stand der Technik getrieben werden muss.

So wurden für die Untersuchungen beispielsweise Kondensationsprodukte von Anilin mit Formaldehyd (MDA) verwendet, die im Wasserstrahlvakuum lediglich ausdestilliert worden waren und deren Anilingehalt demzufolge den 10- bis 20fachen Wert von Produkten aufwies, die zusätzlich einer Wasserdampfdestillation unterworfen worden waren.

Gemäss 2. Ausführungsform des erfindungsgemässen Verfahrens wird das gemäss d) erhaltene Lösungsmittelkondensat entsprechend den Mengenströmen der Aminlösung (i) und Phosgenlösung (ii) in 2 Teilströme aufgeteilt und als Lösungsmittel zur Herstellung dieser Lösungen wiederverwendet. Das Gewichtsverhältnis dieser Lösungsmittelströme zur Herstellung der Lösungen (i) bzw. (ii) liegt bei beiden Ausführungsformen des erfindungsgemässen Verfahrens zwischen 10 : 1 und 1 : 5.

Gemäss 2. Ausführungsform des erfindungsgemässen Verfahrens wird nun der zur Herstellung der Phosgenlösung (ii) verwendete Teilstrom des gemäss d) erhaltenen Lösungsmittelkondensats unbehandelt eingesetzt und lediglich der zur Herstellung der Aminlösung (i) eingesetzte Teilstrom einer erfindungsgemässen Behandlung unterzogen, wobei auf eine destillative Aufarbeitung des erfindungsgemäss behandelten Teilstroms gegebenenfalls verzichtet werden kann. Hierdurch wird ebenfalls erreicht, dass aufgrund der fortlaufenden Überführung eines Teils der Isocyanatgruppen aufweisenden Verbindungen des kontinuierlich im Kreislauf zurückgeführten Lösungsmittels in Isocyanatgruppen-freie Folgeprodukte der Gesamtgehalt des Systems an derartigen Isocyanatverbindungen ein tolerierbares Maximum nicht übersteigt. Die durch Reaktion der Isocyanatgruppen aufweisenden Verbindungen mit den Verbindungen mit gegenüber Isocyanatgruppen reaktionsfähigen Wasserstoffatomen gebildeten, Urethangruppen und insbesondere Harnstoffgruppen aufweisenden Reaktionsprodukte verbleiben dann, wenn auf die Destillation der erfindungsgemäss behandelten Lösungsmittel verzichtet wird, im letztlich gebildeten Verfahrensprodukt und können gewünschtenfalls bei dessen destillativer Aufarbeitung als Rückstandsprodukte entfernt werden. Es handelt sich bei diesen Folgeprodukten jedoch auf jeden Fall um, im Vergleich zu den leichtflüchtigen Monoisocyanaten, physiologisch unbedenkliche Substanzen, die nur in äusserst geringer Konzentration vorliegen und somit keinen nennenswerten Einfluss auf die Produktqualität haben.

Gemäss 2. Ausführungsform des erfindungsgemässen Verfahrens werden vorzugsweise als Verbindungen mit gegenüber Isocyanatgruppen reaktionsfähigen Wasserstoffatomen die oben beispielhaft genannten primären Amine eingesetzt. Besonders bevorzugt werden solche Polyamine verwendet, wie sie auch jeweils als Rohstoff zur Herstellung des Polyisocyanats verwendet werden.

Gemäss einer ersten Variante der 2. Ausführungsform des erfindungsgemässen Verfahrens erfolgt die erfindungsgemässe Behandlung des NCO-Gruppen tragende Verbindungen enthaltenden Lösungsmittels aus der Destillationsstufe d) in völlig analoger Weise zur ersten Ausführungsform des erfindungsgemässen Verfahrens. Im Gegensatz zu dieser wird sie jedoch mengenmässig beschränkt auf den zur Herstellung der Aminolösung (i) zum Einsatz gelangenden Lösungsmittelteilstrom in einem speziell hierzu vorgesehenen Reaktor. Durch die anschliessende Destillation des behandelten Teilstroms vor der Verwendung als Aminlösungsmittel ergeben sich auch hier die Vorteile der separaten Auschleusung der NCO-Gruppen tragenden Verbindungen in Form ihrer schwerflüchtigen Reaktionsprodukte mit OH- bzw. Amingruppen tragenden Reaktionspartnern.

Gemäss einer zweiten Variante der 2. Ausführungsform des erfindungsgemässen Verfahrens erfolgt die erfindungsgemässe Behandlung des zur Herstellung der Aminlösung (i) zum Einsatz gelangenden Lösungsmittelteilstroms in einem speziell hierzu vorgesehenen Reaktor. Bei dieser Variante kommen als Verbindungen mit gegenüber Isocyanatgruppen reaktionsfähigen Wasserstoffatomen vorzugsweise primäre Amine der oben beispielhaft genannten Art zum Einsatz. Wegen ihrer hohen Reaktionsbereitschaft gegenüber Isocyanatgruppen sind hierbei primäre aliphatische Amine der beispielhaft genannten Art besonderd bevorzugt. Die Verbindungen mit gegenüber Isocyanatgruppen reaktionsfähigen Wasserstoffatomen, d.h. vorzugsweise die beispielhaft genannten primären Amine, kommen bei der zweiten Variante der 2. Ausführungsform des erfindungsgemässen Verfahrens vorzugsweise in einem Äquivalentverhältnis von primären Aminogruppen zu Isocyanatgruppen der abzutrennenden Isocyanatkomponenten von 0,9 : 1 bis 1,2 : 1 zum Einsatz. Falls für die erfindungsgemässe Behandlung das gleiche Polyamin eingesetzt wird, wie es auch als Rohstoff zur Herstellung der Polyisocyanate dient, können selbstverständlich auch grosse Überschüsse des Polyamins eingesetzt werden.

Auch bei der 2. Variante der 2. Ausführungsform des erfindungsgemässen Verfahrens erfolgt die erfindungsgemässe Behandlung innerhalb der o.g. Temperaturbereiche bei einer mittleren Verweilzeit der Reaktionskomponenten im Reaktionspartner von 5 bis 60 min. Der so behandelte Lösungsmittelteilstrom wird dann ohne weitere destillative Aufarbeitung zur Herstellung der Aminlösung (i) eingesetzt.

Gemäss einer 3. Variante der 2. Ausführungsform des erfindungsgemässen Verfahrens wird zur Behandlung des zur Herstellung der Aminlösung (i) eingesetzten Lösungsmittelteilstroms das auch als Ausgangsmaterial zur Herstellung der Polyisocyanate verwendete Polyamin verwendet, indem der zur Herstellung der Aminlösung (i) einzusetzende Lösungsmittelteilstrom ohne weitere Vorbehandlung in einen gleichzeitig der Herstellung der Aminlösung (i) dienenden Reaktor geführt wird. Wegen des hierbei vorliegenden extrem hohen Überschusses von Aminogruppen, werden die Isocyanatgruppen der im Kondensat gemäss d) enthaltenen Verbindungen praktisch quantitativ in Harnstoffgruppen überführt. Dieser 3. Variante der 2. Ausführungsform des erfindungsgemässen Verfahrens liegt somit die überraschende Beobachtung zugrunde, dass auch bei einem Verzicht auf eine aufwendige Kolonnendestillation zwecks Eliminierung der leichtflüchtigen, Isocyanatgruppen aufweisenden Verbindungen eine deutliche Reduzierung des Gehalts des Verfahrensprodukts (Polyisocyanat) an derartigen Verbindungen möglich ist, indem das Kondensat d) als solches zur Herstellung der Ausgangslösungen eingesetzt wird.

Der Vorteil der Varianten der zweiten Ausführungsform besteht darin, dass der destillative Aufwand zur Reinigung des Lösungsmittels gegenüber der 1. Ausführungsform entsprechend der Menge des behandelten Teilstromes reduziert ist (1. Variante) oder gänzlich entfällt (2. und 3. Variante).

Die nachfolgenden Beispiele dienen zur weiteren Erläuterung des erfindungsgemässen Verfahrens.

*Beispiel 1*

a) In einem Reaktor, bestehend aus einer drei Rührkessel aufweisenden Kaskade, wurden bei einer mittleren Verweilzeit von 30 Minuten und einer Temperatur von 130°C kontinuierlich 2500 g/h Chlorbenzol mit einem Gehalt an Phenylisocyanat von 2000 ppm mit 25 g/h einer 18 gew.-%igen Lösung von Tetraethylenglykol in Chlorbenzol (entsprechend einem 10%igen Überschuss an OH-Äquivalenten, bezogen auf NCO-Äquivalente) zur Umsetzung gebracht und anschliessend kontinuierlich in den Sumpf einer Destillationskolonne eingespeist. Das Phenylisocyanat-haltige Chlorbenzol entsprach einem Lösungsmittelkondensat, wie es durch Verdampfen und anschliessendes Kondensieren des Lösungsmittels aus dem Produkt einer technischen Phosgenierung von Anilin/Formaldehyd-Kondensaten anfiel, und das vor dem Verdampfen des Lösungsmittels von Phosgen und Chlorwasserstoff befreit wurde. In dem Sumpf der Kolonne wurde vor Beginn der Einspeisung 1900 g Monochlorbenzol zusammen mit 100 g (0,84 Mol) Phenylisocyanat und 89,6 g (0,92 Mol OH) Tetraethylenglykol vorgelegt und während 2 Stunden bei 130°C ohne Destillation umgepumpt.

Mit Beginn der Einspeisung wurde bei leicht erhöhter Sumpftemperatur ohne Rücklauf (unter Normaldruck) kontinuierlich am Kopf der Kolonne eine, der aus dem Reaktor eingebrachten Menge Monochlorbenzol entsprechende Menge Destillat abgenommen.

Der Gehalt an Phenylisocyanat im Chlorbenzol betrug unter diesen Bedingungen (Laufzeit 10 h) 30 ppm.

b) Durch Verdoppelung des Vorreaktorvolumens wurde bei unveränderten Mengenströmen und Reaktionsbedingungen die Verweilzeit in der vorgeschalteten Reaktionsstufe auf 60' erhöht. Der Gehalt an Phenylisocyanat im Chlorbenzol betrug unter diesen Bedingungen (Laufzeit 10 h) < 10 ppm.

c) Nach beendeter Einspeisung wurde der Sumpf der Destillationskolonne bis auf einen Restgehalt von 15 Gew.-% Monochlorbenzol eingeengt. Auch in dem dabei anfallenden Destillat war der Gehalt an Phenylisocyanat < 10 ppm.

Tetraethylenglykol (d.h. OH-Funktionen) konnten unter den Versuchsbedingungen in den Destillaten von a), b) und c) nicht nachgewiesen werden.

d) Das Sumpfprodukt von c) wurde bis 200°C aufgeheizt, wobei das restliche Monochlorbenzol weitgehend abdestillierte. Anschliessend wurde bei einem Vakuum von 30 Torr bis auf 240°C aufgeheizt, wobei insgesamt 74 g (0,62 M) Phenylisocyanat aufgefangen wurde. Das Sumpfprodukt enthielt wieder reaktionsfähige OH-Gruppen und wurde entsprechend seinem Gehalt an OH-Äquivalenten gemäss a) bzw. b) recyclisiert. Entsprechend einem empirisch ermittelten, unter den Versuchsbedingungen auftretenden, durch Nebenreaktionen bedingten Verlust an OH-Äquivalenten wurde pro Zyklus 10% des Sumpfproduktes von d) ausgetragen und vor der Wiederverwendung durch eine entsprechende Menge an Tetraethylenglykol ersetzt.

*Beispiel 2*

In einem Parallelversuch zu Beispiel 1, bei dem in derselben Apparatur und unter den gleichen Bedingungen die gleiche Menge Monochlorbenzol (2500 g/h) mit einem Phenylisocyanatgehalt von ebenfalls 2000 ppm ohne Zusatz von Tetraethylenglykol und ohne vorgelegtes Phenylisocyanat im Sumpf der Kolonne destilliert wurden, lag der Gehalt an Phenylisocyanat im Destillat bei 800 ppm und stieg während des Versuchs (Laufzeit 10 h) auf ca. 900 ppm an.

*Beispiel 3*

In einem Versuch analog Beispiel 1 und 2 wurde in gleicher Menge und unter gleichen Bedingungen isocyanathaltiges Monochlorbenzol eingesetzt, welches aus einer MDI-Lösungsmittelphosgenierung im Technikumsmassstab stammte und einen NCO-Gehalt von 700 ppm (bezogen auf NCO = MG 42) aufwies. Die NCO-Gruppen tragenden Verunreinigungen bestanden zu ca. 80% aus Phenylisocyanat, den Rest bildeten höhersiedende Komponenten (Isocyanatobenzylchlorid, MDI-Isomere).

Bei einer Verweilzeit von 60' im Vorreaktor bei ansonsten gleicher Versuchsausführung wie im Beispiel 1 und 2 beschrieben, betrug der Gehalt an Isocyanatkomponenten im Destillat < 10 ppm Isocyanat (berechnet auf NCO mit dem Molekulargewicht 42).

*Beispiel 4*

In einem Reaktor mit einer mittleren Verweilzeit von 40' und bei einer Temperatur von 130°C werden gleichzeitig eine Monochlorbenzollösung (2000 g/h) mit einem Phenylisocyanatgehalt von 500 ppm und eine 18%ige Monochlorbenzollösung (10 g/h) von Tetraethylenglykol (10% Überschuss an OH-Äquivalenten) vermischt und kontinuierlich in dem Sumpf einer Destillationskolonne eingespeist, indem vor Beginn der Einspeisung 1000 g Monochlorbenzol zusammen mit 551 g (4,63 M) Phenylisocyanat und 449 g Tetraethylenglykol (4,63 OH-Äquivalenten) 2 Stunden lang 130°C ohne Abdestillieren von Monochlorbenzol umgepumpt worden waren.

Mit Beginn der Einspeisung wurde bei Normaldruck und leicht erhöhter Sumpftemperatur ohne Rücklauf im Kolonnenkopf eine in den Reaktor eingebrachte Chlorbenzollösung entsprechende Menge Destillat entnommen.

(Der Gehalt an Phenylisocyanat in Chlorbenzol betrug unter diesen Bedingungen (Laufzeit 10 h) 25 bis 30 ppm.)

*Beispiel 5*

In einem Parallelversuch zu Beispiel 4, bei dem in derselben Apparatur und unter den gleichen Bedingungen die gleiche Menge Monochlorbenzol (2000 g/h) mit einem Phenylisocyanatgehalt von ebenfalls 500 ppm ohne Zusatz von Tetraethylenglykol und ohne vorgelegtes Phenylisocyanat im Sumpf der Kolonne destilliert wurden, betrug der Gehalt an Phenylisocyanat im Destillat ca. 300 ppm mit steigender Tendenz während des Versuchs (Laufzeit 5 h).

*Beispiel 6*

a) In einer kontinuierlich arbeitenden Versuchsphosgenierapparatur wurden 11,88 kg/h einer 15-gewichtsprozentigen Lösung eines Polyamingemischs der Diphenylmethanreihe mit einem Gehalt von 90 Gew.-% an Diaminodiphenylmethan-Isomeren durch Umsetzung mit (ii) 9,5 kg/h einer 45-gewichtsprozentigen Phosgenlösung in an sich bekannter Weise phosgeniert. Als Lösungsmittel dient Chlorbenzol.

b) Aus dem gemäss a) anfallenden Reaktionsgemisch wird anschliessend der Chlorwasserstoff und das gelöste, überschüssige Phosgen durch Ausblasen mit Stickstoff entfernt.

c) Aus der gemäss b) anfallenden Lösung werden anschliessend 15,3 kg/h Chlorbenzol unter Normaldruck mehrstufig, bis zu einer Sumpftemperatur von 180°C in der letzten Stufe abdestilliert. Das als Destillationsrückstand anfallende rohe Polyisocyanat (2,25 kg/h) wird kontinuierlich ausgetragen.

d) Das durch Kondensation der Chlorbenzoldämpfe gemäss c) erhaltene Chlorbenzol (15,3 kg/h) enthält 700 ppm Isocyanatgruppen (berechnet als NCO mit dem Molekulargewicht 42) in Form von Isocyanatgruppen aufweisenden, leichtflüchtigen Verbindungen.

e) Das Kondensat gemäss d) wird anschliessend in einem Rührreaktor, bestehend aus drei hintereinandergeschalteten 5-l-Rührkesseln kontinuierlich unter Rückfluss mit 150 g/h einer 20-gewichtsprozentigen Lösung von Tetraethylenglykol in Chlorbenzol zur Reaktion gebracht und nach Durchlaufen der Kesselkaskade in den Sumpf einer Destillationskolonne überführt, wo reines Chlorbenzol mit einem NCO-Gehalt von weniger als 10 ppm über Kopf abdestilliert wird. Aus dem Sumpf der Kolonne werden stündlich 120-150 g einer Chlorbenzollösung ausgetragen, die einen Gehalt von ca. 45 Gew.-% an Tetraethylenglykol bzw. an Urethanen aus diesem Glykol und den NCO-Gruppen aufweisenden Verbindungen des Kondensates enthält. Das als Destillat gewonnene reine Chlorbenzol wird in zwei Mengenströme aufgeteilt und zur Bereitung der genannten Polyaminlösung (i) und Phosgenlösung (ii) wiederverwendet.

*Beispiel 7*

Beispiel 6 wird wiederholt mit dem Unterschied, dass

d) das durch Kondensation der gemäss c) anfallenden Dämpfe erhaltene Chlorbenzol, (insgesamt 15,3 kg/h) welches in diesem Beispiel 1050 ppm Isocyanatgruppen in Form von Isocyanatgruppen aufweisenden Verbindungen enthält vor der erfindungsgemässen Behandlung in zwei Teilströme von 10,1 kg/h bzw. 5,2 kg/h aufgeteilt wird. Anschliessend wird gemäss der ersten Variante der zweiten Ausführungsform des erfindungsgemässen Verfahrens verfahren:

f) Während der genannte zweite Teilstrom (5,2 kg/h) ohne Abtrennung der darin enthaltenen NCO-Gruppen aufweisenden Verbindungen zur Bereitung frischer Phosgenlösungen (ii) eingesetzt wird, gelangt der erste Teilstrom (10,1 kg/h) in den in Beispiel 6 genannten Reaktor, wo er, wie in Beispiel 6 beschrieben, mit 150 g/h einer 20-gewichtsprozentigen Lösung von Tetraethylenglykol in Chlorbenzol umgesetzt und nach Durchlaufen der Kesselkaskade in der genannten Destillationskolonne in reines Chlorbenzol mit einem NCO-Gehalt von weniger als 10 ppm und einen, überschüssiges Tetraethylenglykol und Urethane enthaltenden Sumpf zerlegt wird. Das so erhaltene, als Destillat anfallende, reine Chlorbenzol wird zur Herstellung frischer Aminlösung (i) wiederverwendet.

Auch in diesem Beispiel fällt das rohe Polyisocyanat als Sumpfprodukt der Stufe c) in einer Menge von 2,25 kg/h an und wird kontinuierlich ausgetragen.

*Beispiel 8*

Beispiel 7 wird wiederholt, mit dem Unterschied, dass

f) der gemäss d) erhaltene erste Teilstrom (10,1 kg/h), der ebenfalls ca. 1050 ppm an Isocyanatgruppen in Form von Isocyanatgruppen aufweisenden Verunreinigungen enthält, in dem genannten Reaktor unter Rückfluss mit 100 g/h einer 16-gewichtsprozentigen Lösung von Hexamethylendiamin und Chlorbenzol umgesetzt wird. Nach Durchlaufen der Kesselkaskade ist der Gehalt an freien Isocyanatgruppen auf weniger als 10 ppm reduziert. Der so behandelte Teilstrom wird ohne weitere Destillation durch Zugabe von Ausgangsamin (1,78 kg/h) zu einer ca. 15-gewichtsprozentigen Aminlösung (i) vermischt, die erneut der Phosgenierungsreaktion unterzogen wird. Die gemäss dieser zweiten Variante der zweiten Ausführungsform des erfindungsgemässen Verfahrens anfallenden durch Reaktion des Hexamethylendiamins mit den NCO-Gruppen aufweisenden Verbindungen gebildeten Harnstoffderivate werden zusammen mit dem Verfahrensprodukt ausgeschleust und können, gewünschtenfalls, von diesem bei dessen destillativer Aufarbeitung abgetrennt werden.

*Beispiel 9*

Beispiel 7 wird wiederholt, mit dem Unterschied, dass

f) der genannte erste Teilstrom (10,1 kg/h) des, Isocyanatgruppen aufweisende Verbindungen enthaltenden, Chlorbenzols ohne weitere Behandlung zur Herstellung einer ca. 15 Gew.-%igen Aminlösung kontinuierlich mit 1,78 kg/h Ausgangsamin umgesetzt wird. Nach Durchlaufen der als Mischer fungierenden Kesselkaskade ist der Gehalt an freien Isocyanatgruppen auf weniger als 20 ppm reduziert. Die so erhaltene Aminlösung wird direkt der Phosgenierungsreaktion zugeführt.

**Patentansprüche**

1. Verfahren zur Herstellung von Polyisocyanaten durch

a) Umsetzung von (i) Lösungen der den Polyisocyanaten zugrundeliegenden Polyamine in einem inerten Lösungsmittel mit (ii) einer Lösung von Phosgen in einem inerten Lösungsmittel in einer ein- oder mehrstufigen Phosgenierungsreaktion,

b) Abtrennung des überschüssigen Phosgens und des gebildeten Chlorwasserstoffs aus dem gemäss a) erhaltenen flüssigen Reaktionsgemisch,

c) Abtrennung des Lösungsmittels zusammen mit leicht flüchtigen, NCO-Gruppen tragenden Verbindungen aus der gemäss b) erhaltenen Lösung durch eine Verdampfung und Gewinnung des gegebenenfalls einer weiteren destillativen Aufarbeitung zuzuführenden Verfahrensprodukts als Verdampfungsrückstand,

d) Gewinnung eines flüchtige, NCO-Gruppen tragende Verbindungen enthaltenden Lösungsmittels durch Kondensation der gemäss c) anfallenden Dämpfe und Wiederverwendung eines Teils des Kondensats zur Herstellung von Aminlösung (i) und eines zweiten Teils des Kondensats zur Herstellung von Phosgenlösung (ii),

dadurch gekennzeichnet, dass man vor der Wiederverwendung des Kondensats entweder

e) die Gesamtmenge des gemäss d) erhaltenen Kondensats ohne vorhergehende destillative Aufarbeitung mit Verbindungen mit gegenüber Isocyanatgruppen reaktionsfähigen Wasserstoffatomen, die mit dem Lösungsmittel kein Azeotrop bilden, die für die destillative Abtrennung ausreichend höher sieden als das Lösungsmittel selbst, und die ausgewählt sind aus der Gruppe bestehend aus ein- und mehrwertigen Alkoholen, mono- und polyfunktionellen primären und sekundären Aminen und Gemischen derartiger Verbindungen, behandelt und anschliessend destillativ von schwerflüchtigen, durch Reaktion dieser Verbindungen mit gegenüber Isocyanatgruppen reationsfähigen Wasserstoffatomen mit im Kondensat enthaltenen, Isocyanatgruppen aufweisenden Verbindungen gebildeten, Reaktionsprodukten befreit, oder

f) die aus undestilliertem, gemäss d) erhaltenem Kondensat bestehende, zur Herstellung der Aminlösung (i) verwendete Teilmenge des Lösungsmittels mit Verbindungen mit gegenüber Isocyanatgruppen reaktionsfähigen Wasserstoffatomen der unter e) genannten Art behandelt und gegebenenfalls anschliessend das so behandelte Lösungsmittel destillativ von schwerflüchtigen, durch Reaktion dieser Verbindungen mit gegenüber Isocyanatgruppen reaktionsfähigen Wasserstoffatomen mit den Isocyanatgruppen aufweisenden Verbindungen gebildeten, Reaktionsprodukten befreit.

2. Verfahren gemäss Anspruch 1, dadurch gekennzeichnet, dass man als Lösungsmittel Chlorbenzol oder o-Dichlorbenzol verwendet.

3. Verfgahren gemäss Ansprüchen 1 und 2, dadurch gekennzeichnet, dass man zur Behandlung gemäss f) Polyamine der als Ausgangsmaterial zur Herstellung der Polyisocyanate verwendeten Art verwendet.

## Claims

1. Process for the production of polyisocyanates by

a) reacting (i) solutions of the polyamines on which the polyisocyanates are based in an inert solvent with (ii) a solution of phosgene in an inert solvent in a single-stage or multi-stage phosgenation reaction,

b) separating off the excess phosgene and the hydrogen chloride formed, from the liquid reaction mixture obtained according to a),

c) separating off the solvent together with readily volatile compounds carrying NCO groups from the solution obtained according to b) by evaporation and recovering the product of the process in the form of an evaporation residue, which product may have to be passed to a further distillative working-up stage,

d) recovering a solvent containing volatile compounds carrying NCO groups by condensation of the vapours obtained according to c) and re-using a part of the condensate for the preparation of amine solution (i) and a second part of the condensate for the preparation of phosgene solution (ii), characterised in that before re-using the condensate either

e) the total quantity of the condensate obtained according to d) is treated, without being previously worked up by distillation, with compounds containing hydrogen atoms which are reactive towards isocyanate groups, which compounds do not form an azeotrope with the solvent and which, for the distillative separation, have boiling points which are sufficiently higher than that of the solvent itself, and which are selected from the group consisting of mono- and polyhydric alcohols, mono- and polyfunctional primary and secondary amines and mixtures of such compounds, and is then freed, by distillation, from not easily volatilised reaction products formed by the reaction of the aforesaid compounds containing hydrogen atoms which are reactive towards isocyanate groups with compounds having isocyanate groups which are contained in the condensate, or

f) the portion of the solvent which consists of undistilled condensate obtained according to d) and which is used for the preparation of the amine solution (i) is treated with compounds containing hydrogen atoms reactive towards isocyanate groups, of the type mentioned under e), and, optionally, the solvent thus treated is then freed, by distillation, from not easily volatilised reaction products formed by the reaction of the aforesaid compounds containing hydrogen atoms reactive towards isocyanate groups with the compounds containing isocyanate groups.

2. Process according to Claim 1, characterised in that chlorobenzene or o-dichlorobenzene is used as the solvent.

3. Process according to Claims 1 and 2, characterised in that polyamines of the type used as starting material for the production of the polyisocyanates are used for the treatment according to f).

## Revendication

1. Procédé de préparation de polyisocyanates par:

a) la réaction (i) de solutions de polyamines à la base des polyisocyanates dans un solvant inerte avec (ii) une solution de phosgène dans un solvant inerte et au cours d'une réaction de phosgénation à une ou plusieurs étapes,

b) la séparation du phosgène en excès et du chlorure d'hydrogène formé hors du mélange réactionnel liquide obtenu suivant (a),

c) la séparation du solvant conjointement avec des composés aisément volatils comportant des groupes NCO hors de la solution obtenue suivant (b) par une évaporation et l'obtention, comme résidu d'évaporation, du produit du procédé devant éventuellement être acheminé à un autre traitement par distillation,

d) l'obtention d'un solvant contenant des composés volatils comportant des groupes NCO, par condensation des vapeurs obtenues suivant (c) et réutilisation d'une partie du condensat pour préparer la solution d'amine (i), ainsi que d'une deuxième partie du condensat pour préparer la solution de phosgène (ii), caractérisé en ce que, avant la réutilisation du condensat:

e) sans traitement préalable par distillation, on traite la quantité totale du condensat obtenu suivant (d) avec des composés comportant des atomes d'hydrogène réactifs vis-à-vis des groupes isocyanate, qui ne forment aucun azéotrope avec le solvant, qui ont, pour la séparation par distillation, un point d'ébullition suffisamment supérieur à celui du solvant lui-même et qui sont choisis parmi le group comprenant des alcools monovalents et polyvalents, des amines primaires et secondaires monofonctionnelles et polyfonctionnelles, ainsi que des mélanges de ces composés puis, par distillation, on libère cette quantité totale des produits réactionnels de faible volatilité formés par réaction de ces composés comportant des atomes d'hydrogène réactifs vis-à-vis des groupes isocyanate avec des composés comportant des groupes isocyanate et contenus dans le condensat, ou

f) on traite la quantité partielle du solvant constituée du condensat non distillé obtenu suivant (d) et utilisée pour la préparation de la solution d'amine (i) avec des composés comportant des atomes d'hydrogène réactifs vis-à-vis des groupes isocyanate du type mentionné sub (e) et ensuite, on libère éventuellement, par distillation, le solvant ainsi traité des produits réactionnels de faible volatilité formés par la réaction de ces composés comportant des atomes d'hydrogène réactifs vis-à-vis des groupes isocyanate avec des composés comportant des groupes isocyanate.

2. Procédé selon la revendication 1, caractérisé en ce que, comme solvant, on utilise le chlorobenzène ou l'o-dichlorobenzène.

3. Procédé selon les revendications 1 et 2, caractérisé en ce que, pour le traitement suivant (f), on utilise des polyamines du type employé comme matière de départ pour la préparation des polyisocyanates.